(19) Europäisches Patentamt
European Patent Office
Office européen des brevets

(11) Veröffentlichungsnummer: **0 297 378 A2**

(12) **EUROPÄISCHE PATENTANMELDUNG**

(21) Anmeldenummer: **88109768.7**

(22) Anmeldetag: **20.06.88**

(51) Int. Cl.⁴: **C07D 413/04 , C07D 261/14 , A01N 43/80**

(30) Priorität: **29.06.87 JP 159741/87**

(43) Veröffentlichungstag der Anmeldung: **04.01.89 Patentblatt 89/01**

(84) Benannte Vertragsstaaten: **BE CH DE ES FR GB IT LI NL**

(71) Anmelder: **NIHON TOKUSHU NOYAKU SEIZO K.K.**
**Itohpia Nihonbashi Honcho Building 7-1, Nihonbashi Honcho 2-chome**
**Chuo-ku Tokyo 103(JP)**

(72) Erfinder: **Kume, Toyohiko**
**6-7-8, Asahigaoka**
**Hino-shi Tokyo(JP)**
Erfinder: **Goto, Toshio**
**3454-21, Honmachida**
**Machida-shi Tokyo(JP)**
Erfinder: **Kamochi, Atsumi**
**2-24-10, Higashi-Toyoda**
**Hino-shi Tokyo(JP)**
Erfinder: **Yanagi, Akihiko**
**2-1200-1, Ome-shi**
**Tokyo(JP)**
Erfinder: **Yagi, Shigeki**
**1-30-7, Izumi**
**Suginami-ku Tokyo(JP)**
Erfinder: **Miyauchi, Hiroshi**
**39-15, Namiki-cho**
**Hachioji-shi Tokyo(JP)**

(74) Vertreter: **Ernst, Hilmar, Dr. et al**
**Bayer AG Konzernverwaltung RP**
**Patentabteilung**
**D-5090 Leverkusen, Bayerwerk(DE)**

(54) **2,5-Dihydropyrrole.**

(57) Offenbart werden neue 2,5-Dihydropyrrole der Formel (I)

EP 0 297 378 A2

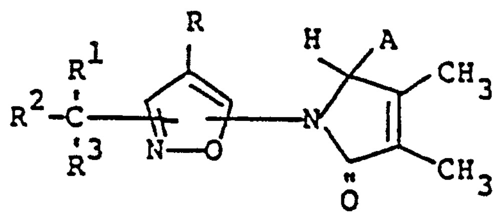

(I)

Verfahren zu ihrer Herstellung und ihre Verwendung als selektive Herbizide sowie ihre Zwischenprodukte der Formel (VIa,b).

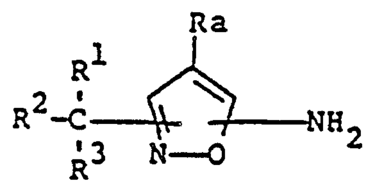

(VIa,b)

## 2,5-Dihydropyrrole

Die vorliegende Erfindung betrifft neue 2,5-Dihydropyrrole, mehrere Verfahren zu ihrer Herstellung und ihre Verwendung als selektive Herbizide.

Es wurde bereits offenbart, daß bestimmte 2,5-Dihydropyrrole als Mittel zur Schädlingsbekämpfung geeignet sind (siehe die JP-OS 23965/1978).

Nunmehr wurden neue 2,5-Dihydropyrrole der Formel (I)

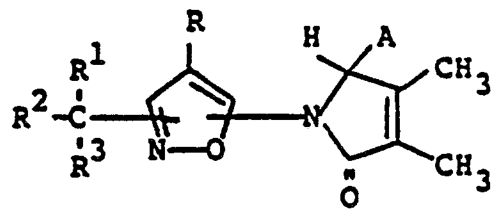

(I)

gefunden, in der

R ein Wasserstoff-Atom, ein Halogen-Atom, eine Alkyl-Gruppe mit 1 bis 6 Kohlenstoff-Atomen, eine Halogenoalkyl-Gruppe mit 1 bis 6 Kohlenstoff-Atomen oder eine Alkoxyalkyl-Gruppe mit insgesamt 2 bis 10 Kohlenstoff-Atomen bezeichnet,

$R^1$, $R^2$ und $R^3$ jeweils ein Wasserstoff-Atom, ein Halogen-Atom, eine Alkyl-Gruppe mit 1 bis 6 Kohlenstoff-Atomen, eine Halogenoalkyl-Gruppe mit 1 bis 6 Kohlenstoff-Atomen oder eine Alkoxyalkyl-Gruppe mit insgesamt 2 bis 10 Kohlenstoff-Atomen bezeichnen oder $R^2$ und $R^3$ zusammen genommen, gemeinsam miit dem Kohlenstoff-Atom, an das sie gebunden sind, einen Kohlenwasserstoff-Ring mit 3 bis 7 Kohlenstoff-Atomen bilden können, mit der Maßgabe, daß $R^1$, $R^2$ und $R^3$ nicht gleichzeitig jeweils ein Wasserstoff-Atom bezeichnen, und

A eine Hydroxyl-Gruppe, eine Gruppe der Formel

$-O-\overset{O}{\overset{\|}{C}}-R^4$, eine Amino-Gruppe, eine Hydroxylamino-Gruppe, eine Alkoxyamino-Gruppe mit 1 bis 4 Kohlenstoff-Atomen, eine O,N-Dialkylhydroxylamino-Gruppe mit insgesamt 2 bis 8 Kohlenstoff-Atomen, eine Mercapto-Gruppe oder ein Halogen-Atom bezeichnet, und

$R^4$ eine Alkyl- oder Halogenoalkyl-Gruppe mit jeweils 1 bis 4 Kohlenstoff-Atomen bezeichnet.

Das Kohlenstoff-Atom in der 2-Position des 2,5-Dihydropyrrol-Rings in den durch die Formel (I) wiedergegebenen Verbindungen der vorliegenden Erfindung ist asymmetrisch. Dementsprechend schließen die 2,5-Dihydropyrrole der Formel (I) gemäß der vorliegenden Erfindung auch optisch aktive Isomere ein.

2,5-Dihydropyrrole der Formel (I) werden enthalten, wenn

a) in dem Fall, in dem A in der Formel (I) Hydroxy ist, Verbindungen der Formel (II),

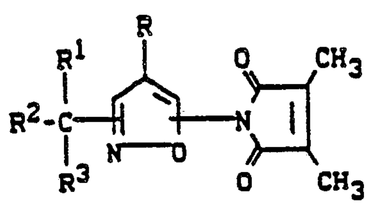

(II)

in der R, $R^1$, $R^2$ und $R^3$ die im Vorstehenden angegebenen Bedeutungen haben, mit einem Reduktionsmittel in Gegenwart inerter Lösungsmittel umgesetzt werden,

oder

b) in dem Fall, in dem A in der Formel (I)

$-O-\overset{O}{\overset{\|}{C}}-R^4$ ist, 2,5-Dihydropyrrole der Formel (Ia)

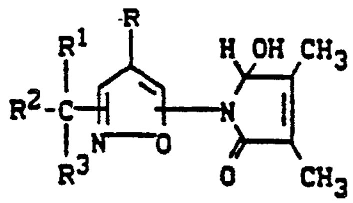

(Ia)

,

in der R, $R^1$, $R^2$ und $R^3$ die im Vorstehenden angegebenen Bedeutungen haben, mit Verbindungen der Formel (III)

$$R^4-\overset{O}{\overset{\|}{C}}-Hal \quad (III),$$

in der $R^4$ die im Vorstehenden angegebenen Bedeutungen hat und Hal ein Halogen-Atom bezeichnet, oder mit Verbindungen der Formel (IV)

$$R^4-\overset{O}{\overset{\|}{C}}-O-\overset{O}{\overset{\|}{C}}-R^4 \quad (IV),$$

in der $R^4$ die im Vorstehenden angegebenen Bedeutungen hat, in Gegenwart inerter Lösungsmittel, gegebenenfalls in Gegenwart säurebindender Mittel, umgesetzt werden

oder

c) in dem Fall, in dem A in der Formel (I) Halogen ist, 2,5-Dihydropyrrole der Formel (Ia)

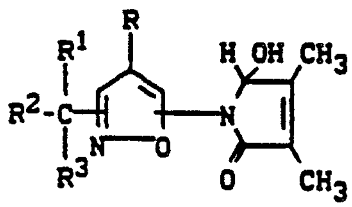

(Ia)

,

in der R, $R^1$, $R^2$ und $R^3$ die im Vorstehenden angegebenen Bedeutungen haben, mit einem Halogenierungsmittel in Gegenwart inerter Lösungsmittel umgesetzt werden,

oder

d) in dem Fall, in dem A in der Formel (I) Iod ist, 2,5-Dihydropyrrole der Formel (Ic)

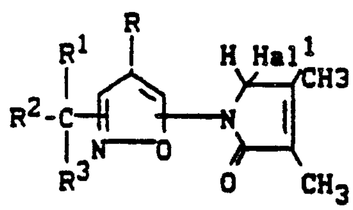

(Ic)

,

in der R, $R^1$, $R^2$ und $R^3$ die im Vorstehenden angegebenen Bedeutungen haben und $Hal^1$ Fluor, Chlor oder Brom bezeichnet, mit Natriumiodid in Gegenwart inerter Lösungsmittel umgesetzt werden,

oder

e) in dem Fall, in dem A in der Formel (I)

$$-\overset{R'}{\overset{|}{N}}-R''$$

ist, worin $R'$ Wasserstoff oder Alkyl mit 1 bis 4 Kohlenstoff-Atomen bezeichnet und $R''$ Wasserstoff, Alkoxy mit 1 bis 4 Kohlenstoff-Atomen oder Hydroxyl bezeichnet, mit der Maßgabe, daß $R'$ nicht Alkyl bezeichnet, wenn $R''$ Wasserstoff oder Hydroxyl bezeichnet, 2,5-Dihydropyrrole der im Vorstehenden angegebenen Formel (Ia)

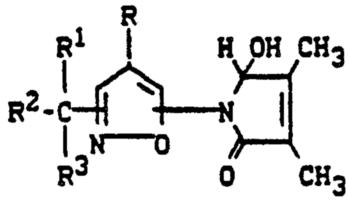

(Ia)

,

in der R, $R^1$, $R^2$ und $R^3$ die im Vorstehenden angegebenen Bedeutungen haben, mit Verbindungen der Formel (V)

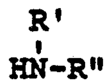

(V)

in denen $R'$ und $R''$ die im Vorstehenden angegebenen Bedeutungen haben, in Gegenwart inerter Lösungsmittel umgesetzt werden,
oder

f) in dem Falle, in dem A in der Formel (I) Mercapto ist, 2,5-Dihydropyrrole der Formel (Id)

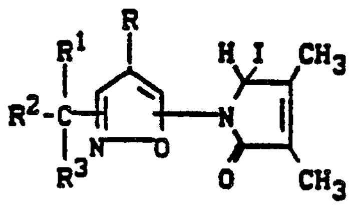

(Id)

,

in der R, $R^1$, $R^2$ und $R^3$ die im Vorstehenden angegebenen Bedeutungen haben, mit Kaliumhydrogen sulfid, Thioharnstoff oder N,N-Dimethylthioformamid in Gegenwart inerter Lösungsmittel umgesetzt werden.

Die neuen 2,5-Dihydropyrrole zeigen potente und selektive herbizide Eigenschaften.

Überraschenderweise zeigen die erfindungsgemäßen 2,5-Dihydropyrrole eine wesentlich größere selektive herbizide Wirkung als diejenigen, die aus dem Stand der Technik, beispielsweise aus der oben angegebenen JP-OS 23965/1978, bekannt sind, und weisen gleichzeitig auch eine gute Verträglichkeit mit Nutzpflanzen auf.

Unter den 2,5-Dihydropyrrolen gemäß der Erfindung sind bevorzugte Verbindungen der Formel (I) diejenigen, in denen

R ein Wasserstoff-Atom, ein Fluor-, Chlor- oder Brom-Atom, eine Alkyl-Gruppe mit 1 bis 4 Kohlenstoff-Atomen, eine Halogenoalkyl-Gruppe mit 1 bis 4 Kohlenstoff-Atomen oder eine Alkoxyalkyl-Gruppe mit insgesamt 2 bis 6 Kohlenstoff-Atomen bezeichnet,

$R^1$, $R^2$ und $R^3$ jeweils ein Wasserstoff-Atom, ein Fluor-, Chlor- oder Brom-Atom, eine Alkyl-Gruppe mit 1 bis 4 Kohlenstoff-Atomen, eine Halogenoalkyl-Gruppe mit 1 bis 4 Kohlenstoff-Atomen oder eine Alkoxyalkyl-Gruppe mit insgesamt 2 bis 6 Kohlenstoff-Atomen bezeichnen und

A eine Hydroxyl-Gruppe, eine Gruppe der Formel

$-O-\overset{O}{\overset{\|}{C}}-R^4$, eine Amino-Gruppe, eine Mercapto-Gruppe oder ein Fluor- oder Chlor-Atom bezeichnet, und

$R^4$ eine Alkyl- oder Halogenoalkyl-Gruppe mit jeweils 1 bis 2 Kohlenstoff-Atomen bezeichnet.

Ganz besonders bevorzugte 2,5-Dihydropyrrole der Formel (I) sind diejenigen, in denen

R eine Wasserstoff-Atom oder eine Methyl-Gruppe bezeichnet,

$R^1$, $R^2$ und $R^3$ jeweils eine Methyl-, Ethyl- oder Chloromethylgruppe bezeichnet und

A eine Hydroxy-Gruppe darstellt.

Speziell erwähnt seien die nachstehenden Verbindungen:

1-(3-tert-Butylisoxazol-5-yl)-3,4-dimethyl-2-hydroxy-5-oxo-2,5-dihydropyrrol,
1-(3-tert-Butyl-4-methylisoxazol-5-yl)-3,4-dimethyl-2-hydroxy-5-oxo-2,5-dihydropyrrol,
1-(5-tert-Butylisoxazol-3-yl)-3,4-dimethyl-2-hydroxy-5-oxo-2,5-dihydropyrrol,
1-[5-(1,1-Dimethyl)propylisoxazol-3-yl)-3,4-dimethyl-2-hydroxy-5-oxo-2,5-dihydropyrrol,
1-(5-tert-Butyl-4-methylisoxazol-3-yl)-3,4-dimethyl-2-hydroxy-5-oxo-2,5-dihydropyrrol und

1-(5-tert-Butylisoxazol-3-yl)-2-chloro-3,4-dimethyl-5-oxo-2,5-dihydropyrrol.

Wenn in dem Verfahren a) beispielsweise 1-(5-tert-Butyl-4-methylisoxazol-3-yl)-3,4-dimethylmaleinimid und Natriumborhydrid als Ausgangsstoffe eingesetzt werden, wird das Verfahren a) durch die folgende Reaktionsgleichung dargestellt:

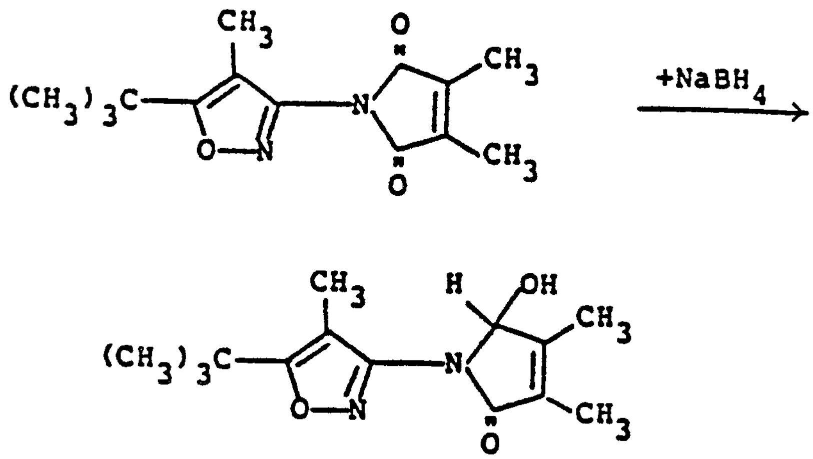


Wenn in dem Verfahren b) beispielsweise 1-(5-tert-Butyl-4-methylisoxazol-3-yl)-2-hydroxy-3,4-dimethyl-5-oxo-2,5-dihydropyrrol und Acetylchlorid als Ausgangsstoffe eingesetzt werden, kann der Reaktionsverlauf durch die folgende Gleichung dargestellt werden:

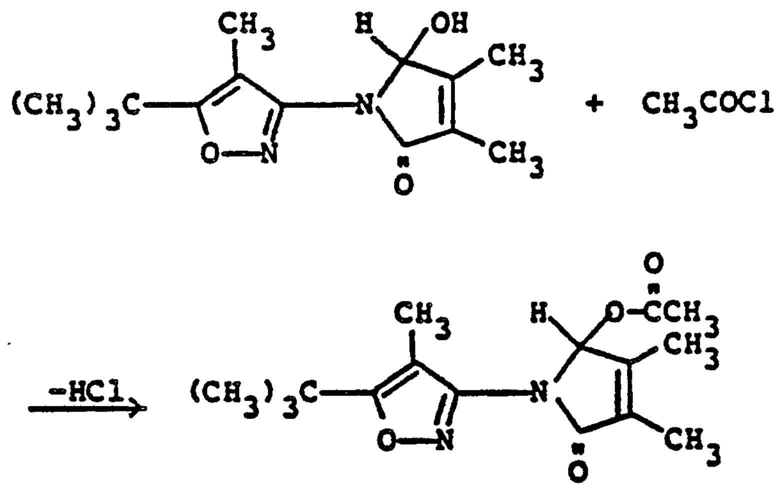


Wenn in dem Verfahren c) beispielsweise 1-(5-tert-Butyl-4-methylisoxazol-3-yl)-2-hydroxy-3,4-dimethyl-5-oxo-2,5-dihydropyrrol und Thionylchlorid als Ausgangsstoffe eingesetzt werden, kann der Reaktionsverlauf durch die folgende Gleichung dargestellt werden:

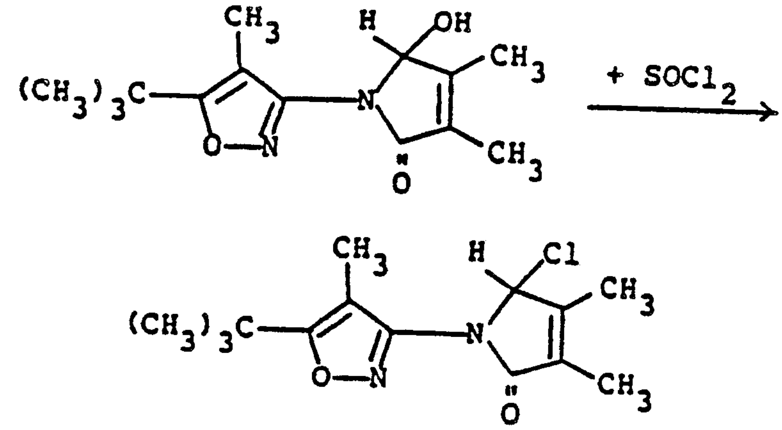


Wenn in dem Verfahren d) beispielsweise 1-(5-tert-Butyl-4-methylisoxazol-3-yl)-2-chloro-3,4-dimethyl-5-oxo-2,5-dihydropyrrol und Natriumiodid als Ausgangsstoffe eingesetzt werden, kann der Reaktionsverlauf durch die folgende Gleichung dargestellt werden:

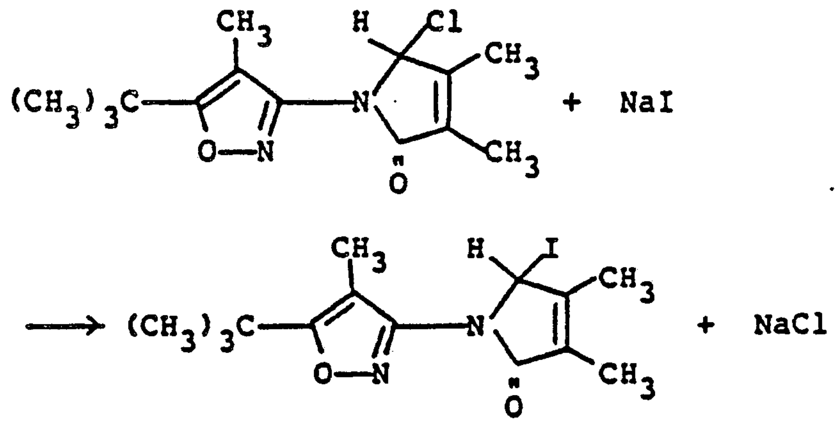


Wenn in dem Verfahren e) beispielsweise 1-(5-tert-Butyl-4-methylisoxazol-3-yl)-2-iodo-3,4-dimethyl-5-oxo-2,5-dihydropyrrol und Ammoniak als Ausgangsstoffe eingesetzt werden, kann der Reaktionsverlauf durch die folgende Gleichung dargestellt werden:

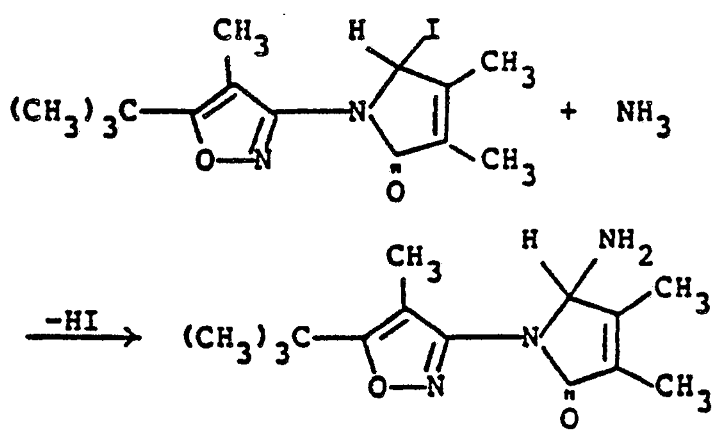


Wenn in dem Verfahren f) beispielsweise 1-(5-tert-Butyl-4-methylisoxazol-3-yl)-2-iodo-3,4-dimethyl-5-oxo-2,5-dihydropyrrol und Kaliumhydrogensulfid als Ausgangsstoffe eingesetzt werden, kann der Reaktionsverlauf durch die folgende Gleichung dargestellt werden:

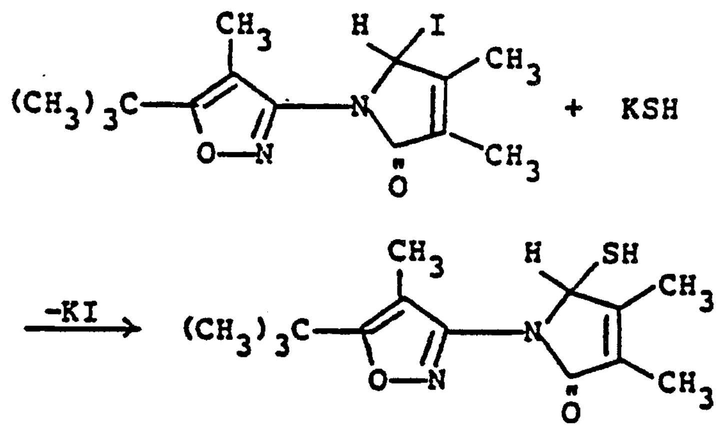

In dem Verfahren a) bezeichnen die Ausgangs-Verbindungen der Formel (II) Verbindungen auf der Basis der oben gegebenen Definitionen für R, R', $R^2$ und $R1^3$, vorzugsweise Verbindungen auf der Basis der oben gegebenen bevorzugten Definitionen.

Die Verbindungen der Formel (II) sind neue Verbindungen und können erhalten werden, wenn Verbindungen der Formel (VI)

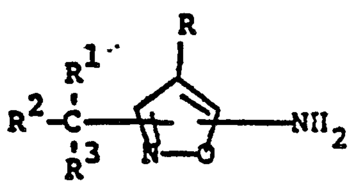

(VI) ,

in der R, R', $R^2$ und $R^3$ die oben angegebenen Bedeutungen haben, mit 2,3-Dimethylmaleinsäureanhydrid der Formel (VII)

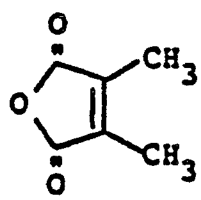

(VII)

umgesetzt werden.

Die Verbindungen der Formel (VI) sind zum Teil bekannt, und beispielsweise sind diejenigen, in denen R ein Wasserstoff-Atom ist, in der JP-OS 81467/1982 beschrieben. Besondere Beispiele sind 3-Amino-5-tert-butyl-isoxazol und 5-Amino-3-tert-butylisoxazol.

In dem Fall, in dem in der Formel (VI) R nicht Wasserstoff ist, sind die Verbindungen der Formel (VI) neu, und die Formel (VI) wird durch die Formel (VIa)ersetzt

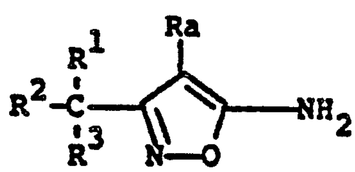

(VIa) ,

in der Ra die oben angegebenen Definitionen für R, in denen R nicht Wasserstoff ist, angibt und R', $R^2$ und $R^3$ die im Vorstehenden angegebenen Bedeutungen haben.

Die vorstehenden Verbindungen der Formel (VIa) können erhalten werden, wenn Verbindungen der Formel (VIII)

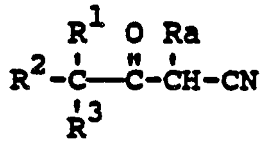
(VIII),

in der Ra, $R^1$, $R^2$ und $R^3$ die im Vorstehenden angegebenen Bedeutungen haben, mit Hydroxylaminhydrochlorid in Gegenwart inerter Lösungsmittel umgesetzt werden.

Die Verbindungen der Formel (VIII) können erhalten werden, wenn Verbindungen der Formel (IX)

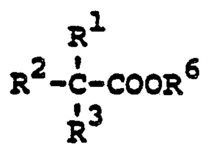
(IX),

in der $R^1$, $R^2$ und $R^3$ die im Vorstehenden angegebenen Bedeutungen haben und $R^6$ eine Niederalkyl-Gruppe bezeichnet, mit Verbindungen der Forml (X)

Ra-$CH_2CN$ (X),

in der Ra die im Vorstehenden angegebenen Bedeutungen hat, in Gegenwart inerter Lösungsmittel umgesetzt werden.

Die Verbindungen der Formeln (IX) und (X) sind bekannt. Beispiele für die Verbindungen der Formel (IX) sind Pivalsinsäureester, und Beispiele für die Verbindungen der Formel (X) sind Propionitril, n-Butyronitril und Isovaleronitril.

In den Fall, in dem in der Formel (VI) R nicht Wasserstoff ist, sind die Verbindungen der Formel (VI) neu, und die Formel (VI) wird durch die Formel (VIb) ersetzt

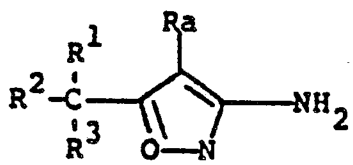
(VIb) ,

in der $R^1$, $R^2$ und $R^3$ und Ra die im Vorstehenden angegebenen Bedeutungen haben.

Die vorstehenden Verbindungen der Formel (VIb) können erhalten werden, wenn die Verbindungen der Formel (XI)

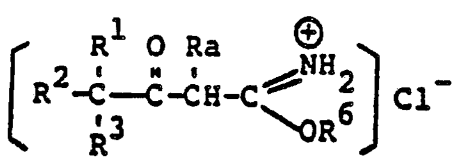
(XI) ,

in der Ra, $R^1$, $R^2$ und $R^3$ und $R^6$ die im Vorstehenden angegebenen Bedeutungen haben, mit Hydroxylaminhydrochlorid umgesetzt werden.

Die Verbindungen der Formel (XI) können erhalten werden, wenn die oben genannten Verbindungen der Formel (VIII) mit Verbindungen der Formel (XII)

$R^6OH$ (XII),

in der $R^6$ die im Vorstehenden angegebenen Bedeutungen hat, unter Einleiten von Hydrogenchlorid umgesetzt werden.

Die Verbindungen der Formel (XII) sind bekannt und schließen beispielsweise Methanol und Ethanol

ein.

Beispiele für das in dem Verfahren a) benutzte Reduktionsmittel sind komplexe Metall-Wasserstoff-Verbindungen wie Natriumborhydrid, Natriumcyanoborhydrid, Diboran, der Boran-Dimethylamin-Komplex, der Boran-Pyridin-Komplex, der Boran-Methylsulfid-Komplex, der Boran-Tetrahydrofuran-Komplex, chirale Aminosäure-Boran-Komplexe, Lithiumaluminiumhydrid und Isobutylaluminiumhydrid.

In dem Verfahren b) bezeichnen die Ausgangs-Verbindungen der Formeln (III) oder (IV) Verbindungen auf der Basis der vorstehenden Definitionen für $R^4$ und Hal, vorzugsweise solche, in denen $R^4$ eine Alkyl- oder Halogenoalkyl-Gruppe mit 1 bis 2 Kohlenstoff-Atomen ist, und Hal ein Chlor-Atom ist.

Acetylchlorid, das sich als spezielles Beispiel für die Formel (III) anführen läßt, ist wohlbekannt. Die Verbindungen der Formel (IV) sind ebenfalls wohlbekannt, und Essigsäureanhydrid sei als spezielles Beispiel genannt.

Das in dem Verfahren c) verwendete Halogenierungsmittel kann beispielsweise Thionylchlorid sein.

Die in dem Verfahren e) eingesetzten Verbindungen der Formel (V) sind wohlbekannt, und ein spezielles Beispiel für sie ist Ammoniak.

Bei der praktischen Durchführung des Verfahrens a) können geeignete Verdünnungsmittel verwendet werden. Sie können beispielsweise sämtliche inerten organischen Lösungsmittel sein.

Beispiele für diese Verdünnungsmittel umfassen Wasser, Alkohole (wie Methanol, Ethanol, n-Propanol, i-Propanol, n-Butanol, sec-Butanol und t-Butanol). Kohlenwasserstoffe (wie Hexan, Benzol und Toluol), halogenierte Kohlenwasserstoffe (wie Dichloromethan und Chloroform), Ether (wie Diethylether, Di-i-propylether, Di-n-propylether, Di-n-butylether Tetrahydrofuran und Dioxan), Amide (wie Dimethylformamid, Dimethylacetamid und 1,3-Dimethyl-2-imidazolidinon) und Sulfoxide (wie Dimethylsulfoxid und Sulfolan).

Das Verfahren a) kann bei Temperaturen innerhalb eines Bereichs von beträchtlicher Breite durchgeführt werden, beispielsweise bei etwa -20 °C bis etwa 80 °C, vorzugsweise bei etwa 0 °C bis etwa 30 °C.

Die Reaktion wird zweckmäßigerweise unter Atmosphärendruck durchgeführt, jedoch ist es auch möglich, unter erhöhtem oder vermindertem Druck zu arbeiten.

Bei der praktischen Durchführung des Verfahrens a) können die gewünschten Verbindungen der Formel (Ia) in einfacher Weise durch Reduzieren der Verbindungen der Formel (II) mit dem oben beispielhaft genannten Reduktionsmittel erhalten werden, wie in den nachstehend angegebenen Beispielen gezeigt wird.

Bei der praktischen Durchführung des Verfahrens b) können geeignete Verdünnungsmittel beispielsweise Ether (wie Dioxan und Tetrahydrofuran), Säureamide (wie Dimethylformamid, Dimethylacetamid und Hexamethylphosphorsäuretriamid) und Sulfoxide (wie Dimethylsulfoxid) sein.

Das Verfahren b) kann in Gegenwart eines säurebindenden Mittels durchgeführt werden. Beispiele für die säurebindenden Mittel sind Hydride, Hydroxide, Carbonate, Hydrogencarbonate und Alkoholate von Alkalimetallen und tertiäre Amine wie Triethylamin, Diethylanilin, Pyridin, 4-Dimethylaminopyridin, 1,8-Diazabicyclo[5.4.0]undecen (DBU) und 1,4-Diazobicyclo[2.2.2]octan (DABCO).

Das Verfahren b) kann bei Temperaturen innerhalb eines Bereichs von beträchtlicher Breite durchgeführt werden, beispielsweise bei etwa -30 °C bis etwa 80 °C, vorzugsweise bei etwa -20 °C bis etwa 50 °C.

Die Reaktion wird zweckmäßigerweise unter Atmosphärendruck durchgeführt, jedoch ist es auch möglich, unter erhöhtem oder vermindertem Druck zu arbeiten.

Bei der praktischen Durchführung des Verfahrens b) können die gewünschten Verbindungen der Formel (Ib) beispielsweise dadurch erhalten werden, daß 1 mol einer Verbindung der Formel (Ia) mit 1 bis etwa 1,5 mol der Verbindung der Formeln (III) oder (IV) in einem inerten Lösungsmittel, gegebenenfalls in Gegenwart des säurebindenden Mittels, umgesetzt wird.

Für die praktische Durchführung des Verfahrens c) können geeignete Verdünnungsmittel Kohlenwasserstoffe (wie Benzol, Toluol und Xylol) und halogenierte Kohlenwasserstoffe (wie Methylenchlorid, Chloroform, Kohlenstofftetrachlorid und Chlorobenzol) sein.

Das Verfahren c) kann bei Temperaturen innerhalb eines Bereichs von beträchtlicher Breite durchgeführt werden, beispielsweise bei etwa -20 °C bis etwa 150 °C, vorzugsweise bei etwa 0 °C bis etwa 80 °C.

Die Reaktion wird zweckmäßigerweise unter Atmosphärendruck durchgeführt, jedoch ist es auch möglich, unter erhöhtem oder vermindertem Druck zu arbeiten.

Bei der praktischen Durchführung des Verfahrens c) können die gewünschten Verbindungen der Formel (Ic) leicht durch Chlorierung einer Verbindung der Formel (Ia), beispielsweise mit Thionylchlorid, erhalten werden.

Bei der praktischen Durchführung des Verfahrens d) können geeignete Verdünnungsmittel alle inerten organischen Lösungsmittel sein.

Beispiele für solche Verdünnungsmittel umfassen aliphatische, alicyclische und aromatische

(gegebenenfalls chlorierte) Kohlenwasserstoffe wie Benzol, Toluol und Xylol; Ether wie Dioxan und Tetrahydrofuran; Ketone wie Aceton, Methylethylketon, Methylisopropylketon und Methylisobutylketon; Nitrile wie Acetonitril, Propionitril und Acrylnitril; Ester wie Ethylacetat und Amylacetat; Säureamide wie Dimethylformamid und Dimethylacetamid und Sulfone und Sulfoxide wie Dimethylsulfoxid und Sulfolan.

Das Verfahren d) kann bei Temperaturen innerhalb eines Bereichs von beträchtlicher Breite durchgeführt werden, beispielsweise bei etwa 0 °C bis etwa 200 °C, vorzugsweise bei etwa 30 °C bis etwa 150 °C. Zweckmäßigerweise wird die Reaktion unter Atmosphärendruck durchgeführt, jedoch ist es auch möglich, unter erhöhtem oder vermindertem Druck zu arbeiten.

Bei der praktischen Durchführung des Verfahrens d) können die gewünschten Verbindungen der Formel (Id) beispielsweise dadurch erhalten werden, daß 1 mol der Verbindung der Formel (Ic) mit 1,5 mol Natriumiodid in einem inerten Lösungsmittel umgesetzt wird.

Bei der praktischen Durchführung des Verfahrens e) können geeignete Verdünnungsmittel alle inerten organischen Lösungsmittel sein.

Beispiele für solche Verdünnungsmittel umfassen Wasser; aliphatische, alicyclische und aromatische (gegebenenfalls chlorierte) Kohlenwasserstoffe wie Hexan, Cyclohexan, Petrolether, Ligroin, Benzol, Toluol, Xylol, Methylenchlorid, Chloroform, Kohlenstofftetrachlorid, Ethylenchlorid und Chlorobenzol; Ether wie Diethylether, Methylethylether, Diisopropylether, Dibutylether, Dioxan und Tetrahydrofuran; Alkohole wie Methanol, Ethanol, Isopropanol, Butanol und Ethylenglycol; und Basen wie Pyridin.

Das Verfahren e) kann bei Temperaturen innerhalb eines Bereichs von beträchtlicher Breite durchgeführt werden, beispielsweise bei etwa 0 °C bis etwa 150 °C, vorzugsweise bei etwa 30 °C bis etwa 100 °C. Die Reaktion wird zweckmäßigerweise unter Atmosphärendruck durchgeführt, jedoch ist es auch möglich, unter erhöhtem oder vermindertem Druck zu arbeiten.

Bei der praktischen Durchführung des Verfahrens e) können die gewünschten Verbindungen der Formel (Ie) beispielsweise dadurch erhalten werden, daß 1 mol der Verbindung der Formel (Ia) mit 2 bis etwa 5 mol der Verbindung der Formel (V) in inerten Lösungsmitteln umgesetzt wird.

Bei der praktischen Durchführung des Verfahrens f) können geeignete Verdünnungsmittel alle inerten organischen Lösungsmittel sein.

Beispiele für solche Verdünnungsmittel umfassen Wasser; aliphatische, alicyclische und aromatische Kohlenwasserstoffe wie Hexan, Cyclohexan, Petrolether, Ligroin, Benzol, Toluol und Xylol; Ether wie Diethylether, Methylethylether, Diisopropylether, Dibutylether, Dioxan und Tetrahydrofuran; Ketone wie Aceton, Methylethylketon, Methylisopropylketon und Methylisobutylketon; Nitrile wie Acetonitril, Propionitril und Acrylnitril; Alkohole wie Methanol, Ethanol, Isopropanol, Butanol und Ethylenglycol; Säureamide wie Dimethylformamid und Dimethylacetamid; Sulfone und Sulfoxide wie Dimethylsulfoxid und Sulfolan; und Basen wie Pyridin.

Das Verfahren f) kann bei Temperaturen innerhalb eines Bereichs von beträchtlicher Breite durchgeführt werden, beispielsweise bei etwa 0 °C bis etwa 200 °C, vorzugsweise bei etwa 30 °C bis etwa 150 °C. Zweckmäßigerweise wird die Reaktion unter Atmosphärendruck durchgeführt, jedoch ist es auch möglich, unter erhöhtem oder vermindertem Druck zu arbeiten.

Bei der praktischen Durchführung des Verfahrens f) können die gewünschten Verbindung der Formel (If) beispielsweise dadurch erhalten werden, daß 1 mol der Verbindung der Formel (Id) mit 1 bis etwa 2 mol Kaliumhydrogensulfid, Thioharnstoff oder N,N-Dimethylthioformamid in inerten Lösungsmitteln umgesetzt wird.

Die erfindungsgemäßen aktiven Verbindungen können als Entlaubungsmittel, Abbrandmittel, Mittel zur Zerstörung breitblättriger Pflanzen und insbesondere als Unkrautvernichtungsmittel eingesetzt werden. Unter Unkräutern im weitesten Sinne sind alle Pflanzen zu verstehen, die an Stellen wachsen, wo sie nicht erwünscht sind. Ob die erfindungsgemäßen Substanzen als totale oder selektive Herbizide wirken, hängt im wesentlichen von der verwendeten Menge ab.

Die aktiven Verbindungen gemäß der Erfindung können beispielsweise in Verbindung mit den folgenden Pflanzen verwendet werden:

<u>Dikotyledonen-Unkräuter der Gattungen:</u>

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver und Centaurea.

Dikotyledonen-Kulturen der Gattungen:

Gossypium, Glycine, Beta, Daucus, Phaseolus, Pisum, Solanum, Linum, Ipomoea, Vicia, Nicotiana, Lycopersion, Arachis, Brassica, Lactuca, Cucumis und Cucurbita.

Monokotyledonen-Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus und Apera.

Monokotyledonen-Kulturen der Gattungen:

Oryza, Zea, Triticum, Hordeum, Avena, Secale, Sorghum, Panicum, Saccharum, Ananas, Asparagus und Allium.

Die Anwendung der erfindungsgemäßen aktiven Verbindungen ist jedoch keineswegs auf diese Gattungen begrenzt, sondern umfaßt auch andere Pflanzen in gleicher Weise.

In Abhängigkeit von den Konzentrationen können die Verbindungen zur totalen Bekämpfung von Unkräutern, beispielsweise auf Industriegeländen und Bahnkörpern sowie auf Wegen und Plätzen mit oder ohne Baumbestand, verwendet werden. Ebenso können die Verbindungen auch zur Unkrautbekämpfung in perennierenden Kulturen, beispielsweise Aufforstungen, Zierbaumpflanzungen, Obstgärten, Weinbergen und -gärten, Zitrushainen, Nußbaumpflanzungen, Bananenplantagen, Kaffeeplantagen, Teeplantagen, Gummiplantagen, Ölpalmenplantagen, Kakaoplantagen, Weichfruchtplantagen und Hopfenfeldern, sowie zur selektiven Unkrautbekämpfung in Jahreskulturen verwendet werden.

Die Wirkstoffe können in übliche Präparate wie Lösungen, Emulsionen, Suspensionen, Pulver, Stäubemittel, Schäume, Pasten, lösliche Pulver, Granulat, Aerosole, mit dem Wirkstoff imprägnierte natürliche und synthetische Materialien, sehr feine Kapseln in polymeren Substanzen, Beschichtungsmittel zum Aufbringen auf das Saatgut und Formulierungen, die in Verbindung mit Verbrennungseinrichtungen wie Räucherpatronen, Räucherdosen und Räucherschlangen eingesetzt werden, sowie ULV-Kaltvernebelungs- und Warmvernebelungspräparate umgewandelt werden.

Diese Präparate können in bekannter Weise hergestellt werden, beispielsweise durch Vermischen der Wirkstoffe mit Streckmitteln, d.h. flüssigen oder verflüssigten gasförmigen oder festen Verdünnungsmitteln oder Trägern, gegebenenfalls unter Verwendung von grenzflächenaktiven Mitteln, d.h. Emulgatoren und/oder Dispergiermitteln und/oder schaumbildenden Mitteln. Bei Verwendung von Wasser als Streckmittel können beispielsweise auch organische Lösungsmittel als Hilfslösungsmittel verwendet werden.

Als flüssige Verdünnungsmittel oder Träger eignen sich hauptsächlich aromatische Kohlenwasserstoffe wie Xylol, Toluol oder Alkylnaphthaline, chlorierte aromatische oder chlorierte aliphatische Kohlenwasserstoffe wie Chlorobenzole, Chloroethylene oder Methylenchlorid, aliphatische oder alicyclische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon, oder stark polare Lösungsmittel wie Dimethylformamid oder Dimethylsulfoxid, sowie auch Wasser.

Unter verflüssigten gasförmigen Verdünnungsmitteln oder Trägern sind Flüssigkeiten zu verstehen, die bei normaler Temperatur und unter normalen Druck gasförmig sein würden, beispielsweise Aerosol-Treibmittel wie halogenierte Kohlenwasserstoffe sowie Butan, Propan, Stickstoff und Kohlenstoffdioxid.

Als feste Träger können gemahlene natürliche Minerale wie Kaoline, Tone, Talkum, Kreide, Quarz, Attapulgit, Montmorillonit oder Diatomeenerde, und gemahlene synthetische Minerale wie hochdisperse Kieselsäure. Aluminiumoxid und Silikate verwendet werden. Als feste Träger für Granulat können zerkleinertes und fraktioniertes natürliches Gesteinsmaterial wie Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengeln verwendet werden.

Als Emulgatoren und/oder Schaumbildner können nichtionische oder anionische Emulgatoren wie

Polyoxyethylen-Fettsäureester, Polyoxyethylen-Fettalkoholether, beispielsweise Alkylarylpolyglycolether, Alkylsulfonate, Alkylsulfate, Arylsulfonate sowie Hydrolyseprodukte des Albumins verwendet werden. Zu den Dispergiermitteln zählen beispielsweise Ligninsulfit-Ablaugen und Methylcellulose.

Haftmittel wie Carboxymethylcellulose und natürliche und synthetische Polymerisate in Form von Pulver, Granulat oder Latices, z.B. Gummi arabicum, Polyvinylalkohol und Polyvinylacetat, können in den Formulierungen verwendet werden.

Es ist möglich, farbgebende Stoffe wie anorganische Pigmente, beispielsweise Eisenoxid, Titanoxid und Preußischbau, und organische Farbstoffe wie Alizarin-Farbstoffe, Azo-Farbstoffe oder Metallphthalocyanin-Farbstoff, und Spurennährstoffe wie Salze von Eisen, Mangan, Bor, Kupfer, Cobalt, Molybdän und Zink, zu verwenden.

Die Präparate enthalten im allgemeinen von 0,1 bis 95 Gewichts-%, vorzugsweise von 0,5 bis 90 Gewichts-% des Wirkstoffs.

Die Wirkstoffe gemäß der vorliegenden Erfindung können als solche oder in Form ihrer Präparate auch zur Unkrautbekämpfung als Mischungen mit anderen Herbiziden eingesetzt werden, wobei Fertigpräparate oder Tankmischungen möglich sind.

Mischungen mit anderen aktiven Verbindungen wie Herbiziden, Fungiziden, Insektiziden, Akariziden, Nematiziden, vogelabweisenden Mitteln, Pflanzennährstoffen sowie Mitteln zur Verbesserung der Bodenstruktur sind ebenfalls möglich.

Die Wirkstoffe können als solche, in Form von Präparaten oder in Gebrauchsformen, die daraus durch weitere Verdünnung hergestellt werden, eingesetzt werden, beispielsweise in Form gebrauchsfertiger Lösungen, Suspensionen, Emulsionen, Pulver, Pasten und als Granulat. Sie werden in üblicher Weise angewandt, beispielsweise durch Bewässern, Sprühen, Zerstäuben oder Streuen.

Die erfindungsgemäßen Wirkstoffe können entweder vor oder nach dem Auflaufen der Pflanzen zur Anwendung gelangen.

Sie können auch vor der Einsaat in den Boden eingearbeitet werden. Insbesondere werden sie nach dem Auflaufen der Pflanzen angewandt.

Die eingesetzten Mengen der aktiven Verbindung können innerhalb eines Bereichs von erheblicher Breite variiert werden. Sie hängen wesentlich von der Art der angestrebten Wirkung ab. Im allgemeinen liegen die aufgewandten Mengen des Wirkstoffs zwischen 0,01 und 4 kg/ha, vorzugsweise zwischen 0,05 und 2 kg/ha, der Bodenoberfläche.

Die Herstellung und Verwendung der erfindungsgemäßen Verbindungen sind den folgenden Beispielen zu entnehmen.

## Herstellungsbeispiele

### Beispiel 1

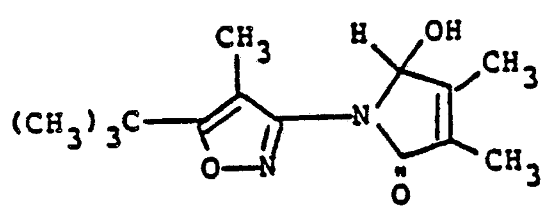

**(Verbindung Nr. 21)**

Natriumborhydrid (0,19 g) wird bei Raumtemperatur unter Rühren nach und nach zu einer methanolischen Lösung (100 ml) von 1-(5-tert-Butyl-4-methylisoxazol-3-yl)-3,4-dimethylmaleinimid (2,54 g) hinzugefügt. Die Mischung wird 3 h bei Raumtemperatur gerührt, und Essigsäure (0,5 g) wird hinzugefügt. Das Lösungsmittel wird unter vermindertem Druck abgedampft. Der Rückstand wird zweimal mit 50 ml

Dichloromethan extrahiert, und der Extrakt wird über wasserfreiem Magnesiumsulfat getrocknet. Abdampfen des Lösungsmittels unter vermindertem Druck lieferte das gewünschte 1-(5-tert-Butyl-4-methylisoxazol-3-yl)-2-hydroxy-3,4-dimethyl-5-oxo-2,5-dihydropyrrol (2,13 g); $n_D^{20}$ = 1,5032.

Beispiel 1A

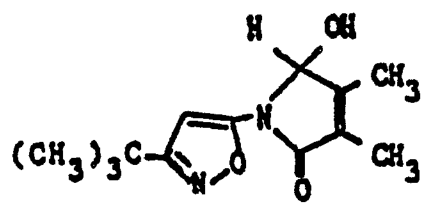

**(Verbindung Nr. 1)**

Zu einer Lösung von 1-(3-tert.-Butyl-isoxazol-5-yl)-3,4-dimethylmaleinimid (1,24g) in Methanol (70ml) gibt man bei Raumtemperatur unter Rühren nach und nach Natriumborhydrid zu (0,11g). Die Mischung wird 3 Stunden bei Raumtemperatur gerührt und anschließend wird Essigsäure (0,3g) zugegeben. Die Lösung wird unter Vakuum eingedampft. Der Rückstand wird mit 50ml Dichlormethan zweimal extrahiert und über wasserfreiem Magnesiumsulfat getrocknet. Nach Abdestillieren des Lösungsmittels unter Druck erhält man das gewünschte 1-(3-tert.-Butylisoxazol-5-yl)-2-hydroxy-3,4-dimethyl-5-oxo-2,5-dihydropyrrol (1,15g).
Schmelzpunkt: 188-189,5° C

Beispiel 2

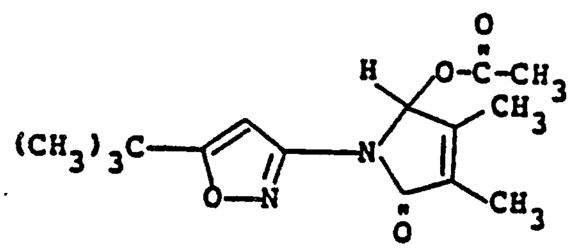

**(Verbindung Nr. 26)**

Eine Dichloromethan-Lösung von Acetylchlorid (0,34 g) wird tropfenweise unter Rühren bei Raumtemperatur zu einer Dichloromethan-Lösung (30 ml) von 1-(5-tert-Butylisoxazol-3-yl)-2-hydroxy-3,4-dimethyl-5-oxo-2,5-dihydropyrrol (1,0 g) und Triethylamin (0,44 g) hinzugefügt. Die Mischung wird 1 h bei Raumtemperatur gerührt. Nach der Reaktion wird das Lösungsmittel unter vermindertem Druck abgedampft. Der Rückstand wird zweimal mit 50 ml Dichloromethan extrahiert und über wasserfreiem Magnesiumsulfat getrocknet. Abdampfen des Lösungsmittels liefert 2-Acetoxy-1-(5-tert-butylisoxazol-3-yl)-3,4-dimethyl-5-oxo-2,5-dihydropyrrol (1,13 g); $n_D^{50}$ = 1,4930.

Beispiel 3

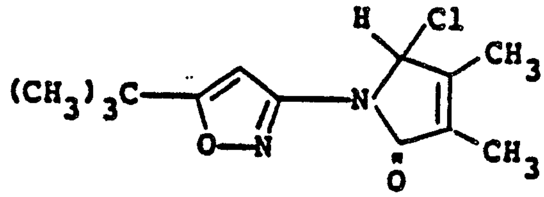

(Verbindung Nr. 27)

Thionylchlorid (0,71 g) wird tropfenweise unter Rühren bei Raumtemperatur zu einer Dichloroethan-Lösung (10 ml) von 1-(5-tert-Butylisoxazol-3-yl)-2-hydroxy-3,4-dimethyl-5-oxo-2,5-dihydropyrrol (1,0 g) hinzugefügt. Die Mischung wird 1 h unter Rückfluß erhitzt. Das Lösungsmittel und das überschüssige Thionylchlorid werden unter vermindertem Druck abgedampft. Der Rückstand wird in Dichloromethan (30 ml) gelöst, und Wasser wird zugegeben. Die Mischung wird mit Kaliumcarbonat alkalisch gemacht und mit Dichloromethan extrahiert. Der Extrakt wird über wasserfreiem Magnesiumsulfat getrocknet, und das Lösungsmittel wird unter vermindertem Druck abgedampft, wonach 1-(5-tert-Butylisoxazol-3-yl)-2-chloro-3,4-dimethyl-5-oxo-2,5-dihydropyrrol (0,54 g) erhalten wurde; Schmp. 115-116 °C.

Verbindungen der vorliegenden Erfindung können mittels der gleichen Verfahren erhalten werden, wie sie im Vorstehenden beschrieben sind. Beispiele sind in den folgenden Tabellen aufgeführt. Tabelle 1 zeigt Verbindungen der folgenden Formel (Ia)

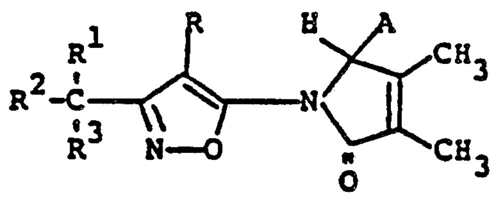

(Ia) ,

die der Formel (I) entsprechen, und Tabelle 2 zeigt Verbindungen der folgenden Formel (Ib)

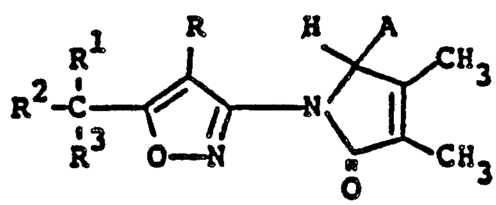

(Ib) ,

die der Formel (I) entsprechen.

## Tabelle 1

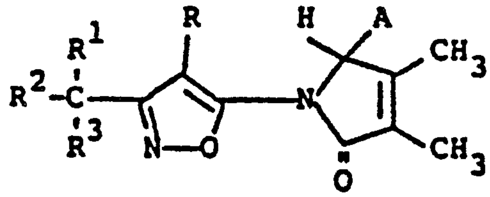

(Ia)

| Verbindung Nr. | R | $R^1$ | $R^2$ | $R^3$ | A | Schmp. °C oder $n_D$ |
|---|---|---|---|---|---|---|
| 1 | H | $CH_3$ | $CH_3$ | $CH_3$ | OH | 188-189.5 |
| 2 | H | $CH_3$ | $CH_3$ | $C_2H_5$ | OH | 108-110 |
| 3 | H | $C_2H_5$ | $C_2H_5$ | $CH_3$ | OH | 99-103 |
| 4 | H | $C_2H_5$ | $C_2H_5$ | $C_2H_5$ | OH | 105-106 |
| 5 | H | $CH_3$ | $CH_3$ | $CH_2Cl$ | OH | 123-130 |
| 6 | H | $CH_3$ | $-(CH_2)_5-$ | | OH | 174-176 |
| 7 | H | $CH_3$ | $CH_3$ | H | OH | 178-180 |
| 8 | H | F | F | F | OH | 175-176 |
| 9 | $CH_3$ | $CH_3$ | $CH_3$ | $CH_3$ | OH | $n_D^{20}$ 1.5090 |
| 10 | $CH_3$ | $CH_3$ | $CH_3$ | $C_2H_5$ | OH | $n_D^{20}$ 1.5109 |
| 11 | $CH_3$ | $C_2H_5$ | $C_2H_5$ | $CH_3$ | OH | $n_D^{20}$ 1.5010 |
| 12 | $CH_3$ | $CH_3$ | $CH_3$ | $CH_2Cl$ | OH | $n_D^{20}$ 1.5135 |
| 13 | $CH_3$ | H | $CH_3$ | $CH_3$ | OH | 115.5-116.5 |
| 14 | $CH_3$ | F | F | F | OH | |
| 15 | $C_2H_5$ | $CH_3$ | $CH_3$ | $CH_3$ | OH | $n_D^{20}$ 1.5102 |
| 16 | Cl | $CH_3$ | $CH_3$ | $CH_3$ | OH | $n_D^{50}$ 1.4874 |

## Tabelle 2

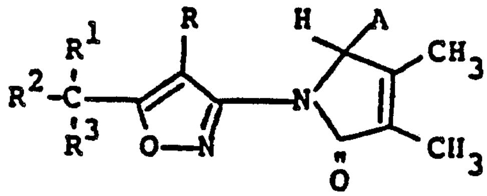

(Ib)

| Verbindung Nr. | R | $R^1$ | $R^2$ | $R^3$ | A | Schmp. °C oder $n_D$ |
|---|---|---|---|---|---|---|
| 17 | H | $CH_3$ | $CH_3$ | $CH_3$ | OH | 147-150 |
| 18 | H | $C_2H_5$ | $CH_3$ | $CH_3$ | OH | 112-113 |
| 19 | H | $C_2H_5$ | $C_2H_5$ | $C_2H_5$ | OH | 106-107 |
| 20 | H | $CH_2Cl$ | $CH_3$ | $CH_3$ | OH | $n_D^{20}$ 1.5185 |
| 21 | $CH_3$ | $CH_3$ | $CH_3$ | $CH_3$ | OH | $n_D^{20}$ 1.5032 |
| 22 | $CH_3$ | $CH_3$ | $CH_3$ | $C_2H_5$ | OH | $n_D^{20}$ 1.5060 |
| 23 | $CH_3$ | $C_2H_5$ | $C_2H_5$ | $CH_3$ | OH | $n_D^{20}$ 1.5072 |
| 24 | $CH_3$ | $CH_3$ | $CH_2Cl$ | $CH_3$ | OH | $n_D^{20}$ 1.4970 |
| 25 | $CH_3$ | H | $CH_3$ | $CH_3$ | OH | $n_D^{20}$ 1.5177 |
| 26 | H | $CH_3$ | $CH_3$ | $CH_3$ | $OC(O)CH_3$ | $n_D^{50}$ 1.4930 |
| 27 | H | $CH_3$ | $CH_3$ | $CH_3$ | Cl | 115-116 |

Beispiel 4

## Synthese eines Zwischenprodukts:

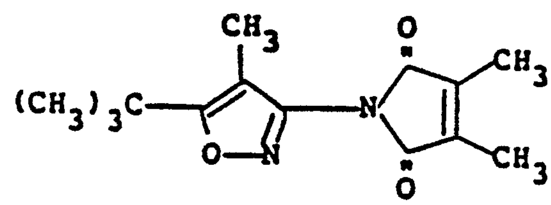

3-Amino-5-tert-butyl-4-methylisoxazol (1,54 g) und 2,3-Dimethylmaleinsäureanhydrid (1,64 g) werden 3 h in Eisessig (30 ml) unter Rückfluß erhitzt. Nach der Reaktion werden Essigsäure und das überschüssige Säureanhydrid abgedampft. Der Rückstand wird in Dichloromethan (40 ml) gelöst, mit 5-proz. Kaliumcarbonat und danach mit Wasser gewaschen und über wasserfreiem Magnesiumsulfat getrocknet. Abdampfen des Lösungsmittels unter vermindertem Druck liefert 1-(5-tert-Butyl)-4-methylisoxazol-3-yl)-3,4-dimethjylmaleinimid (2,54 g); $n_D^{20}$ = 1,5014.

Beispiel 4A

## Herstellung eines Zwischenproduktes:

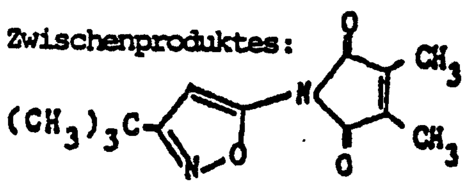

5-Amino-3-tert.-Butylisoxazol (4,21g) und 2,3-Dimethyl-maleinsäureanhydrid (4,16g) werden unter Rückfluß in Eisessig (60ml) 3 Stunden erhitzt. Nach der Reaktion werden der Eisessig und der Überschuß an Säureanhydrid abdestilliert. Der Rückstand wird in Dichlormethan (100ml) gelöst, die Lösung wird zuerst mit 5% Kaliumcarbonat und dann mit Wasser gewaschen und über wasserfreiem Magnesiumsulfat getrocknet.

Nach dem Abdestillieren des Lösungsmittels unter Druck erhält man 1-(3-tert.-Butylisoxazol-5-yl)-3,4-dimethylmaleinimid (6,93g) als viskoses Öl.

Beispiel 5

## Synthese eines Zwischenprodukts:

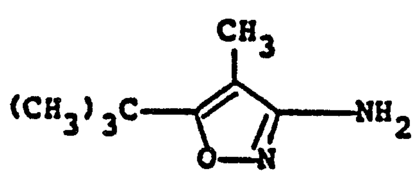

tert-Butyl-1-cyanoethylketon (3,86 g) und wasserfreies Methanol (1,33 g) werden zu wasserfreiem Ether (40 ml) hinzugegeben, und unter Kühlen der Mischung mit einem Eis-Kochsalz-Bad wird Hydrogenchlorid bis zur Sättigung eingeleitet. Man läßt die Mischung 3 h unter Beibehalten der obigen Temperatur und

weiter über Nacht bei Raumtemperatur stehen. Das Lösungsmittel wird unter vermindertem Druck abgedampft, und wasserfreier Ether wird zugegeben, um das Iminoether-hydrochlorid zur Kristallisation zu bringen. Die Kristalle werden durch Filtration gesammelt und unmittelbar anschließend zu einer Methanol-Lösung (40 ml) von Hydroxylamin-hydrochlorid (2,21 g) und Triethylamin (5,61 g) hinzugefügt, und die Mischung wird 1 h unter Rückfluß erhitzt. Nach der Reaktion wird das Lösungsmittel unter vermindertem Druck abgedampft, und Ethanol (30 ml) und konzentrierte Salzsäure (10 ml) werden dem Rückstand zugesetzt. Die Mischung wird 8 h unter Rückfluß erhitzt. Nach der Reaktion wird das Lösungsmittel unter vermindertem Druck abgedampft. Der Rückstand wird mit Kaliumcarbonat alkalisch gemacht, zweimal mit 30 ml Dichloromethan extrahiert und über wasserfreiem Kaliumcarbonat getrocknet. Abdampfen des Lösungsmittels liefert 3-Amino-5-tert-butyl-4-methylisoxazol (3,21 g); Schmp. 66-67,5 °C.

Beispiel 6

## Synthese eines Zwischenprodukts:

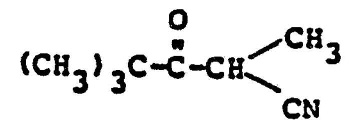

60-proz. Natriumhydrid in Öl (32,0 g) wird zur Entfernung der Öl-Komponente mit trockenem Tetrahydrofuran gewaschen und dann in trockenem Tetrahydrofuran (300 ml) suspendiert. Trockenes Propionitril (27,5 g) und Methylpivalat (55,8 g) werden zu der Suspension hinzugefügt, und die Mischung wird unter Rückfluß erhitzt, bis die Wasserstoff-Entwicklung beendet ist.

Nach der Reaktion läßt man die Reaktionsmischung abkühlen, und das in sehr kleiner Menge verbleibende Natriumhydrid wird mit Methanol zersetzt. Weiterhin wird Wasser (etwa 200 ml) zugegeben, und das Lösungsmittel wird unter vermindertem Druck abgedampft. Der Rückstand wird mit Wasser vermischt und zweimal mit 200 ml Dichloromethan extrahiert, um die unerwünschten Stoffe zu entfernen. Die wäßrige Lösung wird genommen und mit Salzsäure sauer gemacht. Die freigesetzte Öl-Komponente wird dreimal mit 200 ml Dichloromethan extrahiert und über wasserfreiem Magnesiumsulfat getrocknet. Das Lösungsmittel wird unter vermindertem Druck abgedampft. Der Rückstand wird durch Destillation unter vermindertem Druck gereinigt, wonach tert-Butyl-1-cyanoethylketon (45,1 g) erhalten wird;
Sdp. 80-82 °C/24 mbar (18 Torr); $n_D^{20}$ = 1,4085.

Beispiel 7

## Synthese eines Zwischenprodukts:

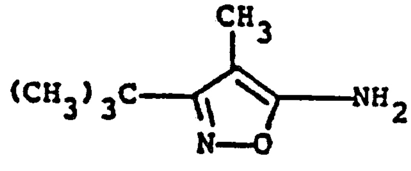

tert-Butyl-1-cyanoethylketon (3,48 g), Hydroxylaminhydrochlorid (2,26 g) und Natriumacetat (3,08 g) werden 3 h in 95-proz. Ethanol (40 ml) unter Rückfluß erhitzt. Nach der Reaktion wird das Lösungsmittel unter vermindertem Druck abgedampft. Der Rückstand wird mit Kaliumcarbonat alkalisch gemacht, zweimal mit 30 ml Dichloromethan extrahiert und über wasserfreiem Kaliumcarbonat getrocknet. Abdampfen des Lösungsmittels unter vermindertem Druck liefert 5-Amino-3-tert-butyl-4-methylisoxazol (3,15 g); $n_D^{30}$ = 1,4935.

Verbindungen der vorliegenden Erfindung, die in den nachstehenden Tabellen aufgeführt sind, können mittels der gleichen Verfahren erhalten werden, wie sie in den vorstehenden Beispielen beschrieben sind.

Tabelle 3 zeigt Verbindungen der folgenden Formel (VIa)

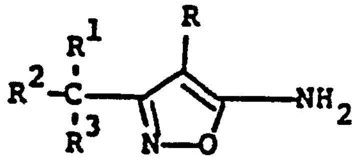

(VIa) ,

und Tabelle 4 zeigt Verbindungen der folgenden Formel (VIb)

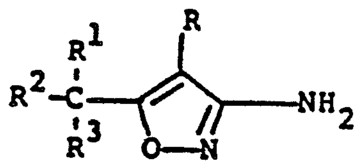

(VIb) .

## Tabelle 3

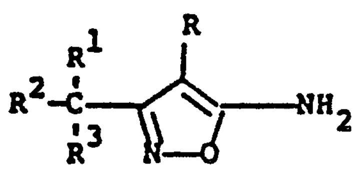

(VIa)

| R | $R^1$ | $R^2$ | $R^3$ | Schmp. °C oder $n_D$ |
|---|---|---|---|---|
| $CH_3$ | $CH_3$ | $CH_3$ | $CH_3$ | $n_D^{30}$ 1.4935 |
| $CH_3$ | $CH_3$ | $CH_3$ | $C_2H_5$ | |
| $CH_3$ | $C_2H_5$ | $C_2H_5$ | $CH_3$ | |
| $CH_3$ | $CH_3$ | $CH_2Cl$ | $CH_3$ | |
| $CH_3$ | H | $CH_3$ | $CH_3$ | 57 - 79 |
| $CH_3$ | F | F | F | |
| $C_2H_5$ | $CH_3$ | $CH_3$ | $CH_3$ | |
| 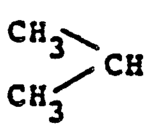 | $CH_3$ | $CH_3$ | $CH_3$ | 159-160 |

## Tabelle 4

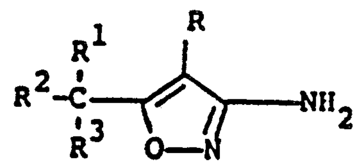 (VIb)

| R | $R^1$ | $R^2$ | $R^3$ | Schmp. °C oder $n_D$ |
|---|---|---|---|---|
| $CH_3$ | $CH_3$ | $CH_3$ | $CH_3$ | 66 - 67.5 |
| $CH_3$ | $CH_3$ | $CH_3$ | $C_2H_5$ | |
| $CH_3$ | $C_2H_5$ | $C_2H_5$ | $CH_3$ | |
| $CH_3$ | $CH_2Cl$ | $CH_3$ | $CH_3$ | |
| $CH_3$ | $CH_3$ | H | $CH_3$ | |
| $C_2H_5$ | $CH_3$ | $CH_3$ | $CH_3$ | |
| 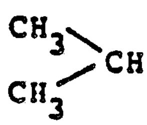 | $CH_3$ | $CH_3$ | $CH_3$ | |

Biologische Tests

Vergleichsverbindung:

**E-1:**

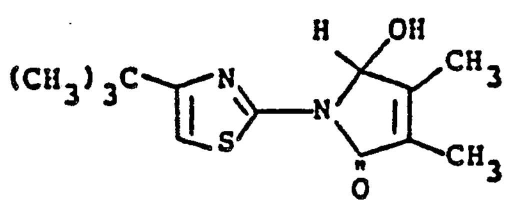

(die in der JP-OS 23965/1978 beschriebene Verbindung).

Beispiel 8

Test mittels Bodenbehandlung vor dem Auflaufen:

Herstellung eines Wirkstoff-Präparats

Träger: 5 Gewichtsteile Aceton;
Emulgator: 1 Gewichtsteil Benzyloxypolyglycolether.

Eine Formulierung der Test-Verbindung in Form eines emulgierbaren Konzentrats wird erhalten durch Vermischen von 1 Gewichtsteil der aktiven Verbindung mit den oben angegebenen Mengen des Trägers und des Emulgators. Eine vorher festgelegte Menge der Formulierung wird mit Wasser verdünnt, um eine Test-Chemikalie zu erhalten.

Test-Verfahren

In einem Gewächshaus wurden Mais-Samen in Töpfe (500 $cm^2$), die mit Ackererde gefüllt waren, eingesät, und Erde, die Samen von Gänsefuß (Chenopodium album L.), Hühnerhirse (Echinocloa crus-galli) und wildem grauen Amaranth (Aramanthus blitum) enthielt, wurde über die Mais-Samen in einer Tiefe von 1 cm gestreut.

Einen Tag nach der Einsaat wurde eine vorher festgelegte Menge der Test-Chemikalie gleichmäßig über die Oberflächenschicht des Bodens in jedem der Test-Töpfe gesprüht.

Vier Wochen nach dem Sprühen wurden die herbizide Wirkung und der Grad der Phytotoxizität gegenüber den Nutzpflanzen (Mais) geprüft und mit Hilfe der folgenden Skalen von 0 bis 5 bewertet:

Die herbizide Wirkung wurde als prozentuales Verhältnis der Unkrautvernichtung, bezogen auf eine unbehandelte Fläche, nach der folgenden Skala bewertet:

5 weingstens 95 % (verdorrt)
4 wenigstens 80 %, jedoch weniger als 95 %
3 wenigstens 50 %, jedoch weniger als 80 %
2 wenigstens 30 %, jedoch weniger als 50 %
1 wenigstens 10 %, jedoch weniger als 30 %
0 weniger als 10 % (unwirksam)

Die Phytotoxizität gegenüber Mais wurde als Phytotoxizitätsrate, bezogen auf die unbehandelte Fläche, nach der folgenden Skala bewertet:

5 wenigstens 90 % (Ausrottung)
4 wenigstens 50 %, jedoch weniger als 90 %
3 wenigstens 30 %, jedoch weniger als 50 %
2 wenigstens 10 %, jedoch weniger als 30 %
1 mehr als 0 %, jedoch weniger als 10 %
0 0 % (keine Phytotoxizität)

Die Test-Ergebnisse sind anhand typischer Beispiele in Tabelle 5 aufgeführt.

Tabelle 5

| Verbindung Nr. | Wirkstoff-Menge kg/ha | Herbizide Wirkung | | | Phytotoxizität gegen Mais |
|---|---|---|---|---|---|
| | | A | B | C | |
| 9 | 0,5<br>0,25 | 5<br>5 | 5<br>5 | 5<br>4 | 0<br>0 |
| 17 | 0,5<br>0,25 | 5<br>5 | 5<br>5 | 5<br>5 | 1<br>0 |
| Vergleichs-Verbindung | | | | | |
| E-1 | 0,5<br>0,25 | 1<br>0 | 1<br>0 | 0<br>0 | 0<br>0 |
| Anmerkung | | | | | |
| A: Wilder grauer Amaranth<br>B: Gänsefuß<br>C: Hühnerhirse | | | | | |

Beispiel 9

Test auf höher gelegenem Ackerboden mittels Laub-Behandlung:

In einem Gewächshaus wurden Mais-Samen in Töpfe (500 $cm^2$), die mit Ackererde gefüllt waren, eingesät, und Erde, die Samen von Hühnerhirse und Gänsefuß enthielt, wurde in einer Tiefe von 1 cm darüber gestreut.

Nach der Einsaat wurden die Pflanzen 14 Tage angezogen, und eine vorher festgelegte Menge einer Test-Chemikalie, die wie in Beispiel 8 hergestellt worden war, wurde gleichmäßig über die Pflanzen in den Töpfen gesprüht.

Vier Wochen nach dem Sprühren wurden die herbizide Wirkung und die Phytotoxizität gegenüber den Nutzpflanzen (Mais) anhand der gleichen Bewertungsskalen wie in Beispiel 8 geprüft. Die Ergebnisse sind anhand typischer Beispiele in Tabelle 6 aufgeführt.

Tabelle 6

| Verbindung Nr. | Wirkstoff-Menge kg/ha | Herbizide Wirkung | | Phytotoxizität gegen Mais |
|---|---|---|---|---|
| | | C | B | |
| 21 | 0,5<br>0,25 | 5<br>5 | 5<br>5 | 0<br>0 |
| Vergleichs-Verbindung | | | | |
| E-1 | 0,5<br>0,25 | 2<br>1 | 2<br>1 | 0<br>0 |
| Anmerkung | | | | |
| C und B sind die gleichen, wie sie in der Fußnote zu Tabelle 5 bezeichnet sind. | | | | |

**Ansprüche**

1. 2,5-Dihydropyrrole der Formel (I)

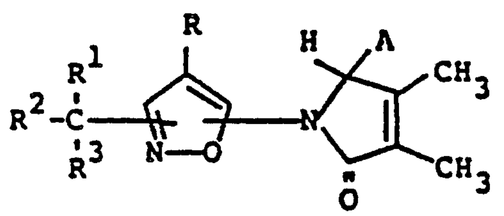 (I) ,

in der
R ein Wasserstoff-Atom, ein Halogen-Atom, eine Alkyl-Gruppe mit 1 bis 6 Kohlenstoff-Atomen, eine Halogenoalkyl-Gruppe mit 1 bis 6 Kohlenstoff-Atomen oder eine Alkoxyalkyl-Gruppe mit insgesamt 2 bis 10 Kohlenstoff-Atomen bezeichnet,
$R^1$, $R^2$ und $R^3$ jeweils ein Wasserstoff-Atom, ein Halogen-Atom, eine Alkyl-Gruppe mit 1 bis 6 Kohlenstoff-Atomen, eine Halogenoalkyl-Gruppe mit 1 bis 6 Kohlenstoff-Atomen oder eine Alkoxyalkyl-Gruppe mit insgesamt 2 bis 10 Kohlenstoff-Atomen bezeichnen oder $R^2$ und $R^3$ zusammen genommen, gemeinsam mit dem Kohlenstoff-Atom, an das sie gebunden sind, einen Kohlenwasserstoff-Ring mit 3 bis 7 Koblenstoff-Atomen bilden können, mit der Maßgabe, daß $R^1$, $R^2$ und $R^3$ nicht gleichzeitig jeweils ein Wasserstoff-Atom bezeichnen, und
A eine Hydroxyl-Gruppe, eine Gruppe der Formel
$-O-\overset{O}{\overset{\|}{C}}-R^4$, eine Amino-Gruppe, eine Hydroxylamino-Gruppe, eine Alkoxyamino-Gruppe mit 1 bis 4 Kohlenstoff-Atomen, eine O,N-Dialkylhydroxylamino-Gruppe mit insgesamt 2 bis 8 Kohlenstoff-Atomen, eine Mercapto-Gruppe oder ein Halogen-Atom bezeichnet, und
$R^4$ eine Alkyl- oder Halogenoalkyl-Gruppe mit jeweils 1 bis 4 Kohlenstoff-Atomen bezeichnet.

2. Verbindungen nach Anspruch 1, dadurch gekennzeichnet, daß
R ein Wasserstoff-Atom, ein Fluor-, Chlor- oder Brom-Atom, eine Alkyl-Gruppe mit 1 bis 4 Kohlenstoff-Atomen, eine Halogenoalkyl-Gruppe mit 1 bis 4 Kohlenstoff-Atomen oder eine Alkoxyalkyl-Gruppe mit insgesamt 2 bis 6 Kohlenstoff-Atomen bezeichnet,
$R^1$, $R^2$ und $R^3$ jeweils ein Wasserstoff-Atom, ein Fluor-, Chlor- oder Brom-Atom, eine Alkyl-Gruppe mit 1 bis 4 Kohlenstoff-Atomen, eine Halogenoalkyl-Gruppe mit 1 bis 4 Kohlenstoff-Atomen oder eine Alkoxyalkyl-Gruppe mit insgesamt 2 bis 6 Kohlenstoff-Atomen bezeichnen und
eine Hydroxyl-Gruppe, eine Gruppe der Formel

$-O-\overset{O}{\overset{\|}{C}}-R^4$, eine Amino-Gruppe, eine Mercapto-Gruppe oder ein Fluor- oder Chlor-Atom bezeichnet, und
$R^4$ eine Alkyl- oder Halogenoalkyl-Gruppe mit jeweils 1 bis 2 Kohlenstoff-Atomen bezeichnet.

3. Verbindungen nach Anspruch 1, dadurch gekennzeichnet, daß
R ein Wasserstoff-Atom oder eine Methyl-Gruppe bezeichnet,
$R^1$, $R^2$ und $R^3$ jeweils eine Methyl-, Ethyl- oder Chloromethylgruppe bezeichnet und
A eine Hydroxy-Gruppe darstellt.

4. Verfahren zur Herstellung von 2,5-Dihydropyrrolen der Formel (I)

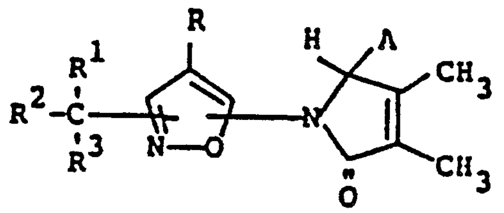

(I) ,

in der
R ein Wasserstoff-Atom, ein Halogen-Atom, eine Alkyl-Gruppe mit 1 bis 6 Kohlenstoff-Atomen, eine Halogenoalkyl-Gruppe mit 1 bis 6 Kohlenstoff-Atomen oder eine Alkoxyalkyl-Gruppe mit insgesamt 2 bis 10 Kohlenstoff-Atomen bezeichnet,
$R^1$, $R^2$ und $R^3$ jeweils ein Wasserstoff-Atom, ein Halogen-Atom, eine Alkyl-Gruppe mit 1 bis 6 Kohlenstoff-Atomen, eine Halogenoalkyl-Gruppe mit 1 bis 6 Kohlenstoff-Atomen oder eine Alkoxyalkyl-Gruppe mit insgesamt 2 bis 10 Kohlenstoff-Atomen bezeichnen oder $R^2$ und $R^3$ zusammen genommen, gemeinsam mit dem Kohlenstoff-Atom, an das sie gebunden sind, einen Kohlenwasserstoff-Ring mit 3 bis 7 Kohlenstoff-Atomen bilden können, mit der Maßgabe, daß $R^1$, $R^2$ und $R^3$ nicht gleichzeitig jeweils ein Wasserstoff-Atom bezeichnen, und
A eine Hydroxyl-Gruppe, eine Gruppe der Formel
$-O-\overset{O}{\overset{\|}{C}}-R^4$, eine Amino-Gruppe, eine Hydroxylamino-Gruppe, eine Alkoxyamino-Gruppe mit 1 bis 4 Kohlenstoff-Atomen, eine O,N-Dialkylhydroxylamino-Gruppe mit insgesamt 2 bis 8 Kohlenstoff-Atomen, eine Mercapto-Gruppe oder ein Halogen-Atom bezeichnet, und
$R^4$ eine Alkyl- oder Halogenoalkyl-Gruppe mit jeweils 1 bis 4 Kohlenstoff-Atomen bezeichnet,
dadurch gekennzeichnet, daß

a) in dem Fall, in dem A in der Formel (I) Hydroxy ist, Verbindungen der Formel (II),

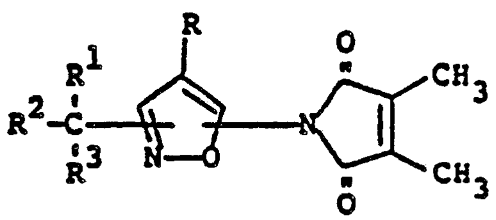

(II) ,

in der R, $R^1$, $R^2$ und $R^3$ die im Vorstehenden angegebenen Bedeutungen haben, mit einem Reduktionsmittel in Gegenwart inerter Lösungsmittel umgesetzt werden,
oder

b) in dem Fall, in dem A in der Formel (I)
$-O-\overset{O}{\overset{\|}{C}}-R^4$ ist, 2,5-Dihydropyrrole der Formel (Ia)

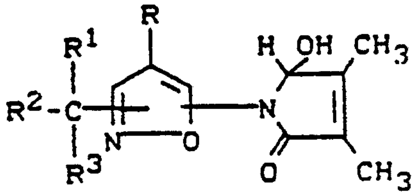

(Ia)

,

in der R, $R^1$, $R^2$ und $R^3$ die im Vorstehenden angegebenen Bedeutungen haben, mit Verbindungen der Formel (III)

$R^4-\overset{O}{\overset{\|}{C}}-Hal$ (III),

in der $R^4$ die im Vorstehenden angegebenen Bedeutungen hat und Hal ein Halogen-Atom bezeichnet, oder mit Verbindungen der Formel (IV)

$R^4-\overset{O}{\overset{\|}{C}}-O-\overset{O}{\overset{\|}{C}}-R^4$ (IV),

in der $R^4$ die im Vorstehenden angegebenen Bedeutungen hat, in Gegenwart inerter Lösungsmittel, gegebenenfalls in Gegenwart säurebindender Mittel, umgesetzt werden,

oder

c) in dem Fall, in dem A in der Formel (I) Halogen ist, 2,5-Dihydropyrrole der Formel (Ia)

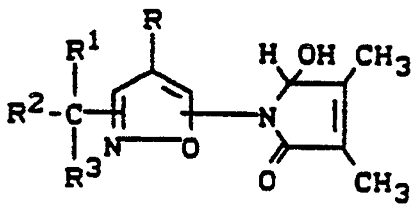

(Ia)

,

in der R, $R^1$, $R^2$ und $R^3$ die im Vorstehenden angegebenen Bedeutungen haben, mit einem Halogenierungsmittel in Gegenwart inerter Lösungsmittel umgesetzt werden,

oder

d) in dem Fall, in dem A in der Formel (I) Iod ist, 2,5-Dihydropyrrole der Formel (Ic)

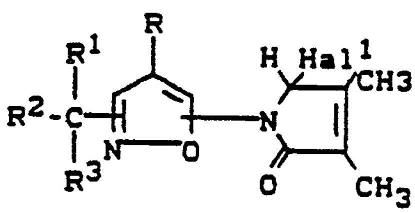

(Ic)

,

in der R, $R^1$, $R^2$ und $R^3$ die im Vorstehenden angegebenen Bedeutungen haben und $Hal^1$ Fluor, Chlor oder Brom bezeichnet, mit Natriumiodid in Gegenwart inerter Lösungsmittel umgesetzt werden,

oder

e) in dem Fall, in dem A in der Formel (I)

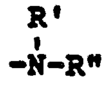

ist, worin $R'$ Wasserstoff oder Alkyl mit 1 bis 4 Kohlenstoff-Atomen bezeichnet und $R''$ Wasserstoff, Alkoxy mit 1 bis 4 Kohlenstoff-Atomen oder Hydroxyl bezeichnet, mit der Maßgabe, daß $R'$ nicht Alkyl bezeichnet, wenn $R''$ Wasserstoff oder Hydroxyl bezeichnet, 2,5-Dihydropyrrole der im Vorstehenden angegebenen Formel (Ia)

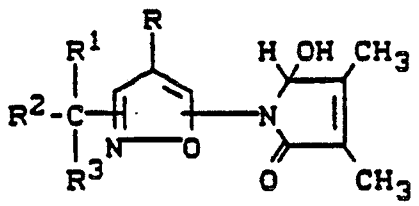

(Ia)

,

in der R, $R^1$, $R^2$ und $R^3$ die im Vorstehenden angegebenen Bedeutungen haben, mit Verbindungen der Formel (V)

$$HN(R')-R''$$ (V)

in denen $R'$ und $R''$ die im Vorstehenden angegebenen Bedeutungen haben, in Gegenwart inerter Lösungsmittel umgesetzt werden,
oder

f) in dem Falle, in dem A in der Formel (I) Mercapto ist, 2,5-Dihydropyrrole der Formel (Id)

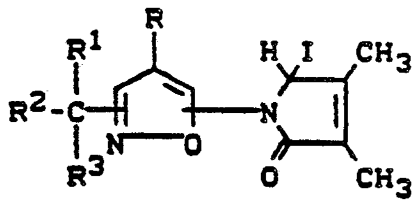

(Id)

,

in der R, $R^1$, $R^2$ und $R^3$ die im Vorstehenden angegebenen Bedeutungen haben, mit Kaliumhydrogensulfid, Thioharnstoff oder N,N-Dimethylthioformamid in Gegenwart inerter Lösungsmittel umgesetzt werden.

5. Herbizide Zusammensetzungen, dadurch gekennzeichnet, daß sie wenigstens ein 2,5-Dihydropyrrol der Formel (I) nach den Ansprüchen 1 bis 4 enthalten.

6. Verfahren zur Bekämpfung von Unkräutern, dadurch gekennzeichnet, daß 2,5-Dihydropyrrole der Formel (I) nach den Ansprüchen 1 bis 4 auf Unkräuter und/oder deren Lebensraum zur Einwirkung gebracht werden.

7. Verwendung von 2,5-Dihydropyrrolen der Formel (I) nach den Ansprüchen 1 bis 4 zur Bekämpfung von Unkräutern.

8. Verfahren zur Herstellung herbizider Zusammensetzungen, dadurch gekennzeichnet, daß 2,5-Dihydropyrrole der Formel (I) nach den Ansprüchen 1 bis 4 mit Streckmitteln und/oder grenzflächenaktiven Mitteln vermischt werden.

9. Aminoisooxazole der Formel (VIa,b)

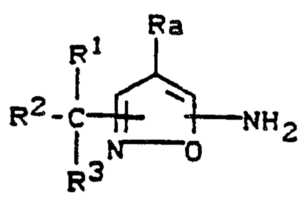

(VIa,b)

,

in der
Ra ein Halogen-Atom, eine Alkyl-Gruppe mit 1 bis 6 Kohlenstoff-Atomen, eine Halogenoalkyl-Gruppe mit 1 bis 6 Kohlenstoff-Atomen oder eine Alkoxyalkyl-Gruppe mit insgesamt 2 bis 10 Kohlenstoff-Atomen bezeichnet und
$R^1$, $R^2$ und $R^3$ jeweils ein Wasserstoff-Atom, ein Halogen-Atom, eine Alkyl-Gruppe mit 1 bis 6 Kohlenstoff-Atomen, eine Halogenoalkyl-Gruppe mit 1 bis 6 Kohlenstoff-Atomen oder eine Alkoxyalkyl-Gruppe mit insgesamt 2 bis 10 Kohlenstoff-Atomen bezeichnen oder

$R^2$und $R^3$ zusammen genommen, gemeinsam mit dem Kohlenstoff-Atom, an das sie gebunden sind, einen Kohlenwasserstoff-Ring mit 3 bis 7 Kohlenstoff-Atomen bilden können,
mit der Maßgabe, daß $R^1$, $R^2$ und $R^3$ nicht gleichzeitig jeweils ein Wasserstoff-Atom bezeichnen.

10. Verfahren zur Herstellung von Aminoisoxazolen der Formel (VIa,b)

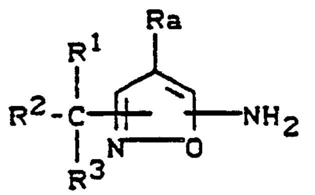

(VIa,b)

in der
Ra ein Halogen-Atom, eine Alkyl-Gruppe mit 1 bis 6 Kohlenstoff-Atomen, eine Halogenoalkyl-Gruppe mit 1 bis 6 Kohlenstoff-Atomen oder eine Alkoxyalkyl-Gruppe mit insgesamt 2 bis 10 Kohlenstoff-Atomen bezeichnet und
$R^1$, $R^2$ und $R^3$ jeweils ein Wasserstoff-Atom, ein Halogen-Atom, eine Alkyl-Gruppe mit 1 bis 6 Kohlenstoff-Atomen, eine Halogenoalkyl-Gruppe mit 1 bis 6 Kohlenstoff-Atomen oder eine Alkoxyalkyl-Gruppe mit insgesamt 2 bis 10 Kohlenstoff-Atomen bezeichnen oder
$R^2$ und $R^3$ zusammen genommen, gemeinsam mit dem Kohlenstoff-Atom, an das sie gebunden sind, einen Kohlenwasserstoff-Ring mit 3 bis 7 Kohlenstoff-Atomen bilden können,
mit der Maßgabe, daß $R^1$, $R^2$ und $R^3$ nicht gleichzeitig jeweils ein Wasserstoff-Atom bezeichnen,
dadurch gekennzeichnet, daß
Verbindungen der Formel (VIII)

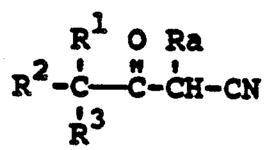

(VIII),

in der Ra, $R^1$, $R^2$ und $R^3$ die im Vorstehenden angegebenen Bedeutungen haben, oder Verbindungen der Formel (XI)

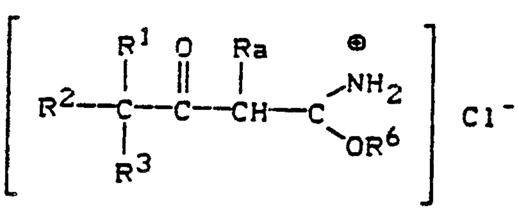

(XI)

in der Ra, $R^1$, $R^2$, $R^3$ und $R^6$ die im Vorstehenden angegebenen Bedeutungen haben, mit Hydroxylamin-hydrochlorid umgesetzt werden, gegebenenfalls in Gegenwart inerter Verdünnungsmittel.